# EUROPEAN PATENT SPECIFICATION

(11) **EP 4 452 376 B1**
(45) Date of publication and mention of the grant of the patent: **21.10.2026**
(21) Application number: 22840198.0
(22) Date of filing: 22.12.2022
(51) Int. Cl.: A61M 16/04, A61M 16/20

(54) **SYSTEM FOR AVOIDING LEAKAGE IN ENDOTRACHEAL TUBE WITH SINGLE OR DOUBLE CUFF**
SYSTEM ZUR VERMEIDUNG VON LECKAGEN IN EINEM ENDOTRACHEALTUBUS MIT EINZELNER ODER DOPPELTER MANSCHETTE
SYSTÈME POUR ÉVITER UNE FUITE DANS UNE SONDE D'INTUBATION ENDOTRACHÉALE AVEC UN BALLONNET UNIQUE OU DOUBLE

(30) Priority: 22.12.2021 US 202163292838 P
(43) Date of publication of application: 30.10.2024
(73) Proprietor: Medical Technology for Life, 9320 Aalst (BE)
(72) Inventor: BRAEM, Kristof, 9320 Nieuwerkerken - Aalst (BE)
(74) Representative: Arnold & Siedsma
(86) International application number: PCT/EP2022/087589
(87) International publication number: WO 2023/118494

(56) References cited:
- WO-A1-99/66975
- WO-A2-2007/149301
- CA-A- 995 542
- FR-A1- 2 196 819
- NL-A- 9 101 495
- US-A- 4 979 505
- US-A1- 2011 073 115
- US-A1- 2016 101 253

## Description

### TECHNICAL FIELD

This disclosure concerns a special design or configuration of cuffs, e.g. single or double cuff, for endotracheal (ET) tubes, such that an improved sealing of cuffs with the patient's tracheal wall being achieved, and hence air or fluid leakage being avoided. The improved sealing is not only better performing, it is also more comfortable and less harming for the patient, causing no risk of permanent damage to the patient's inner body. The special cuff configuration comprises a multi-section cloud shape and may further be provided with one or more one-directional valves, all or not being inflatable.

This disclosure is additionally related to sensing means in an ET tube double cuff configuration, in particular detecting and/or measuring whether or not cuffs are well-sealing with the patient's trachea wall, or in other words, if a leakage occurs there in between.

### BACKGROUND

Conventionally, a single cuff is inflated with air following insertion of the ET tube into human mid-trachea to achieve an airtight seal of the space between the tube and surrounding tracheal wall. Alternative tubes with double cuff also exist and were introduced as a possible solution for several defects in conventional single cuff endotracheal tubes. One of these defects is for example leakage on the cuff because of insufficient cuff pressure, or by changing situation of the patient and performing manipulations. Hence, secretions may pass through, subsequently accumulating in the upper trachea. In addition, serious - possibly permanent - injury of the tracheal wall may occur due to high lateral-wall-cuff pressure being applied in case of a single cuff in order have a non-leak seal. However, it turns out that, in practice, the double cuff is not always experienced as most efficient nor appropriate solution, and the defects, although maybe partially reduced, remain present.

As such the cuffed endotracheal tube has been routinely employed for many decades to prevent upper airway obstruction or to facilitate artificial ventilation of the unconscious or anesthetized patient. Three basic problems can arise with ET tubes provided with single or double cuff. For each of these problems, solutions were proposed in the past but making another problem sometimes worse, or either causing new problems. The first basic problem is related to air leaking out of the trachea during inspiration, for which an increased cuff pressure can help but you need to know if and when you will reach dangerous pressures hampering (blood) perfusion, or even producing tissue necrosis. Not knowing when the filled cuffs are giving the necessary air tight seal, will result most probably in higher cuff pressure than needed. The second basic problem is the opposite, and concerns fluid leaking into the trachea mostly during expiration, also referred to as silent aspiration. Higher cuff pressures have only limited effect here, but cuff material and/or shape as well as subglottic aspiration are more important but can be quite complicated and therefore not useful for short interventions. The third basic problem regards the mucosal ischemia being more problematic when applying a higher cuff pressure.

Recent studies have shown that the ET tubes on the market today, whether single cuffed, or with double cuffs, are still showing several defects. Specific cuff designs have been introduced to make improvements in ET tubes, amongst which for instance the Microcuff^{®} can be mentioned, featuring an advanced micro-thin polyurethane cuff and being specifically designed for the paediatric airway. The Taperguard^{™} cuff on the other hand, is featuring a tapered conical-like cuff. A major drawback of current specific cuff designs, is their level of complexity and difficulty to make, hence in general being rather expensive as compared to standard cuffs.

The challenge we are still facing, is that we need to search for each individual patient and for each mode of ventilation and airway management the lowest possible cuff pressures, while avoiding air and/or fluid leaking, and if/when/where leakage still would occur having a good knowledge thereof in order to react quickly and appropriately. Moreover, when pressure needs to be elevated, this should be controlled and kept as short as possible in time. A simple response for all-round and frequent use in ventilation systems is needed.

An aim of the embodiments of this disclosure is to guarantee an air and fluid tight-fit seal between the cuffs of the endotracheal tube and the tracheal wall of the patient, with the lowest possible cuff pressure, optionally being automatically regulated. The air and fluid tight-fit seal with the lowest possible cuff pressure will minimize any possible injury caused on the tracheal walls due to a minimum inflation of the cuffs, ensuring no leakage or a leak-free ventilation and avoiding silent aspiration of oral fluids, and as such any secretions from passing through. In particular, the aim is to provide an air and fluid tight-fit seal even during high-pressure positive ventilation, like during lung recruitment, as well as during active aspiration.

It is also an aim of the embodiments of this disclosure to detect and/or measure whether or not cuffs are well-sealing with the patient's trachea wall, or in other words, if a leakage occurs there in between. The document US 2011/073115 A1 represents relevant prior art for the present invention.

### SUMMARY

According to a first aspect, the present disclosure provides a cloud cuff for an endotracheal tube (ETT) system, according to claim 1, said ETT system comprising a ventilation tube and one or more cuff inflation lumens (also referred to as cuff inflation lines). In case of e.g. one cuff inflation lumen or cuff inflation line, such lumen or line may be in fluid or fluidic communication with an interior portion of the cuff, and may be used to inflate and/or deflate the cuff. The name cloud cuff is derived from the special cloud shape the cuff has. Instead of cloud cuff, we may also refer to stepped, lobed or ribbed cuff, whereas this cloud shape also resembles like being stepped, lobed or ribbed. The cloud cuff being attached or attachable to the ventilation tube of the ETT system, can be seen as a continuous multi-section (or multi-chamber) envelope to be inflated when the ETT system has been intubated into a patient. The multi-section cloud cuff being inflatable, comprises two or more sections (hence also being inflatable) being connected. The sections can be seen as physically connected, meaning that a new section starts where former one ends, or else, whereas each section has two ends, one of the ends of a section is connected with one of the ends of another (adjacent) section. The sections can also be seen as connected through air (because of the hollow shape of the cuff when inflated).

Whereas a standard cylindrical cuff having a constant diameter along most of its length when fully inflated, the diameter can now change or vary (along cuff length) per cuff section, and the diameter can reach a local maximum per section. The cloud cuff can be seen as comprising different diameters when inflated. At least two sections are having a different (local) maximum diameter. Adjacent sections may have a common diameter where they connect or meet. This difference in maximum diameter is generally rather slight. At least one section operates as sealing portion, and may have e.g. the largest diameter, however having a smaller diameter is also possible. When a typical cylindrical cuff (known in the art) is inserted into a patient's trachea and inflated, the cuff walls of the cylinder are unable to inflate to their maximum diameter and may fold in on themselves in order to fit in the trachea, which causes wrinkles and leak paths to form. The sealing portion or sealing section in accordance with the invention is adapted to form a wrinkle-free band against a patient's tracheal wall when inflated, wherein the wrinkle-free sealing band or section is configured to prevent leakage of fluid or air passing the wrinkle-free sealing band when inflated. By means of example, when having a cloud cuff with three sections, and being inflated, one section touches the tracheal wall wrinkle-free, and a smaller section (i.e. having a smaller diameter) does not touch the tracheal wall at all.

The sections can be referred to as stepped, lobe-like or rib-like sections. Each section may have a gradually changing or varying diameter in that each section (having two ends) can have a first end with a first diameter, a second end with a second diameter, and a middle (part) being determined by the (local) maximum diameter, hence being larger than both said first and second diameter. This being a direct result or consequence of the fact that the sections here, when inflated, all have a convex surface. First and second diameter can be different, i.e. the first diameter can be smaller or larger than the second diameter. Possibly, the first and second diameter are the same. When inflated, the cloud cuff can have a frustro-conical shape, in case the cuff comprises at least two sections, or the cloud cuff can have a (stepped) double tapered shape, in case the cuff comprises at least three sections. The cloud cuff may comprise polyethylene terephthalate (PETP), low-density polyethylene (LDPE), polyvinyl chloride (PVC), silicone, neoprene, polyisoprene, polypropylene, or polyurethane (PU). The ETT system may comprise a distal end adapted to be inserted into a patient's trachea and a proximal end adapted to be connected to a ventilator.

According to an embodiment, the cloud cuff is configured to be inflated to cuff pressures of 5-30 cm H₂O, exceptionally to cuff pressures up to 100 cm H₂O.

According to an embodiment, the cloud cuff is configured such that one or more sections make each minor contact with the tracheal mucosa, such that ischemia phenomena being reduced. The cuff has to be small and narrow, compressing the mucosa (membrane) along a ring being narrower than a tracheal cartilage ring. The contact of a standard cuff that is made with the tracheal mucosa, is in general over a ring distance of about 3cm along the length of the cuff. The cloud cuff may be configured such that each section makes minor contact with the tracheal mucosa, over a ring distance of less than 2 cm, preferably less than 1 cm, along the length of the cuff, herewith providing perfusion in between the different sections. In other words, the sections can be designed as small enough to achieve this minor contact, but also to have sufficient space in the transition between adjacent sections due to their different diameters and/or their gradually changing diameters.

According to an embodiment, the two or more sections are defined as separate (or individual) balloons stacked together (like for example weights in a dumbbell). Whereas the sections being inflatable, the balloons are also inflatable. The balloons may be physically connected by their stacking, but may also be connected through air by either having only one cuff inflation lumen (or cuff inflation line) for inflating each of them, or else via an opening provided between adjacent balloons. Each of the balloons may be connected separately to respective and corresponding intramural channels in the ventilation tube allowing independent inflation of the balloons, and for example these intramural channels being cuff inflation lumens. A balloon is to be understood to the result of inflating an in essence 2D surface material configured for enveloping a volume. Typically a balloon has one input for inflating but the balloon concept is not restricted thereto as multiple inputs are possible also as outlined above.

Two one-directional valves (which are inflatable) are provided in the vicinity of the cloud cuff. The valves will in case of, possibly sudden and/or short but high, underpressure or overpressure make contact with the patient's tracheal wall, such that additional sealing being provided. In other words, the contact is made that strong, such that additional sealing takes place. This underpressure or overpressure is typically temporarily, because occurring at inspiration or expiration (e.g. from 0.5 to 3 seconds), and thereafter dropping/levelling back to zero. Regardless of the cuff pressure (e.g. 20 mbar), a good sealing with or against the tracheal wall is ensured, due to temporal overpressure (e.g. 40 mbar, 50 mbar) of the ventilator or underpressure (e.g. 20 mbar, 30 mbar, 50 mbar, or even 100 mbar) of the aspiration device.

According to an embodiment, in between two sections, one of the one-directional valves is provided (at proximal end of the cuff), which will in case of underpressure make contact with the patient's tracheal wall, herewith achieving additional sealing in proximal position, and/or in between (other) two sections, another one of the one-directional valves being provided (at distal end of the cuff), which will in case of overpressure make contact with the patient's tracheal wall, herewith achieving additional sealing in distal position. The provision of the valves in between two sections of the cuff, may be chosen for achieving easier folding/unfolding of the valves. In case of the valve at overpressure, and causing additional sealing in proximal position, the valve will typically direct in a direction towards the patients' mouth. In case of the valve at underpressure (typically another valve than the one at overpressure), and causing additional sealing in distal position, the valve will typically direct in the opposite direction towards the patients' lungs. The design or configuration of the one-directional valve will lead to functioning in one direction or the other (opposite) one. According to an embodiment, a valve could be designed or configured to work or function in the two opposing directions, hence a two-directional valve.

An inflatable one-directional valves is provided in the vicinity of the cloud cuff, preferably adjacent to the two or more sections thereof, which will when inflated make contact with the patient's tracheal wall, such that additional sealing being provided. Whereas the cuff having two ends (proximal and distal), an inflatable one-directional valve is provided adjacent to the sections of both ends, i.e. one at proximal and one at distal end. The inflatable one-directional valve(s) can be incorporated in the cloud cuff, such that for example we have one inflatable whole of valve(s) and cuff sections. These configurations wherein the one-directional valve is provided onto or in the vicinity of a cuff for an endotracheal tube (ETT) system, define a valve-cuff arrangement within the different embodiments herein provided.

It is noted that the one-directional valve(s) could also be applicable to a standard single cuff (or double cuff). Whereas the valves may be inflatable, they may provide standard fitting/closure. The one-directional valves, especially the inflatable ones, could be interpreted as a cuff with a specific form or shape, not only intended for closure, but also functioning as one-directional valve for overpressure or underpressure.

According to a second aspect, the present disclosure provides an ETT system for ventilating a patient, comprising a ventilation tube, one or more cuff inflation lumens or lines, and a cloud cuff comprising two or more sections being attached or attachable to the ventilation tube as in the first aspect. The sections being connected, wherein at least two sections having a (slight) difference in (local) maximum diameter, such that at least one section operates as sealing portion, adapted to form a wrinkle-free band against a patient's tracheal wall when inflated, wherein the wrinkle-free sealing band is configured to prevent leakage of fluid or air passing the wrinkle-free sealing band when inflated. The ETT system for ventilating a patient, may further comprise one or more one-directional valves in the vicinity of the cloud cuff, e.g. a one-directional valve in between two sections thereof, which will in case of underpressure make contact with the patient's tracheal wall, herewith achieving additional sealing in proximal position, and/or another one-directional valve in between (other) two sections, which will in case of overpressure make contact with the patient's tracheal wall, herewith achieving additional sealing in distal position.

According to a third aspect, the present disclosure provides an ETT system for ventilating a patient, comprising of two cloud cuffs as in the first aspect, comprising a primary cloud cuff and a secondary cloud cuff in distal position with respect to said primary cloud cuff, said two cloud cuffs being provided with one or more cuff inflation lumens or lines to inflate and/or deflate said two cuffs, and an inter-cuff region, connecting said primary cloud cuff and secondary cloud cuff. In case of e.g. one cuff inflation lumen, such lumen may be in fluid communication with an interior portion of each cuff, and may be used to inflate and/or deflate each cuff. The cuffs are then serially connected via the cuff inflation lumen. The inter-cuff region may be provided with means for sensing and/or measuring airflow parameters (e.g. amount of airflow, airway pressure or air leakage). The cuff pressures can be controlled or regulated to achieve the lowest possible pressures while having no leak as measured in the inter-cuff region. By measuring whether there is a (air or fluid) leak or not in the inter-cuff region, one automatically knows what the minimum pressure is needed to have a good seal. A one-directional valve (all or not inflatable) may be provided with the primary cloud cuff and will in case of underpressure make contact with the patient's tracheal wall, herewith achieving additional sealing in proximal position, whereas another one-directional valve (all or not inflatable) may be provided with the secondary cloud cuff and will in case of overpressure make contact with the patient's tracheal wall, herewith achieving additional sealing in distal position.

Disclosed but unclaimed is a method for ventilating a patient, comprising (i) providing an ETT system as in the third aspect; (ii) inserting said ETT system orally into the patient such that said two cloud cuffs are placed into the trachea of the patient; (iii) inflating said two cloud cuffs including said inter-cuff region; (iv) sensing and/or measuring one or more airflow parameters in said inter-cuff region; and (v) further/additionally inflating said two cloud cuffs in case of (sudden/significant) change (e.g. decrease or drop) of (one of) the one or more airflow parameters such that a constant pressure in said inter-cuff region (and/or said two cloud cuffs) being achieved.

These and other features, aspects, and advantages will be better understood with reference to the following description and the appended claims.

Additional features and advantages of the embodiments described herein will be set forth in the detailed description that follows, and in part will be readily apparent to those skilled in the art from that description or recognized by practicing the embodiments described herein, including the detailed description that follows, the claims, as well as the appended drawings.

It is to be understood that both the foregoing general description and the following detailed description describe various embodiments and are intended to provide an overview or framework for understanding the nature and character of the claimed subject matter, and should not be considered in isolation but can be independently combined. The accompanying drawings are included to provide a further understanding of the various embodiments, and are incorporated into and constitute a part of this specification. The drawings illustrate the various embodiments described herein, and together with the description serve to explain the principles and operations of the claimed subject matter.

### BRIEF DESCRIPTION OF THE DRAWINGS

FIGS. 1A and 1B illustrate an endotracheal tube in accordance with the art, in FIG. 1A being intubated in a patient, and in FIG. 1B either with inflated and uninflated cuff.
FIGS. 2A, 2B, and 2C illustrate an embodiment of a cuff configuration for a single cuff ET tube in inflated state, whereas FIG. 2D illustrates an embodiment of the single cuff configuration in uninflated state.
FIG. 3 illustrates another embodiment of a cuff configuration for a single cuff ET tube.
FIGS. 4A, 4B, and 4C illustrate an embodiment of a cuff configuration for a double cuff ET tube.
FIGS. 5A and 5B illustrate another embodiment of a cuff configuration for a double cuff ET tube.
FIGS. 6A and 6B illustrate yet another embodiment of a cuff configuration for a double cuff ET tube.
FIGS. 7A and 7B illustrate a further embodiment of a cuff configuration for a double cuff ET tube.
FIGS. 8A and 8B illustrates in FIG. 8A an embodiment of a type of double cuff ET tube comprising a double cuff configuration, wherein the tube being provided with sensing and/or measuring means, for detecting if leakage occurs between the cuffs and the patient's tracheal wall, including in FIG. 8B a cuff controller to be connected therewith.
FIGS. 9A and 9B illustrates in FIG. 9A a picture embodiment of a cuff configuration for a single cuff ET tube, wherein the single cuff is shown in uninflated state, including in FIG. 9B a picture embodiment of the same single cuff configuration, now shown in fully inflated state.
FIG. 10A and 10B illustrates in FIG. 10A a picture embodiment of a cuff configuration for a single cuff ET tube, inserted into a patient's trachea, wherein the single cuff is shown in very little inflated state, including in FIG. 10B a picture embodiment of the same single cuff ET tube in the patient's trachea, now shown with the single cuff in fully inflated state.
FIG. 11 illustrates a picture embodiment of the same single cuff ET tube as in FIG. 10A and 10B, inserted in the patient's trachea, shown with the single cuff in fully inflated state, and wherein secretions are blocked or prevented from moving further in the trachea.

### DETAILED DESCRIPTION

The present disclosures relates to the improved intubation state of patients, based on the application of a new type of endotracheal (ET) tube comprising of one or two cuffs. The present disclosure particularly relates to a cuff configuration for the single or double cuff ET tube. The cuffs are specifically configured for achieving an improved sealing with the patient's tracheal wall, herewith avoiding air and/or fluid leakage. Although being tight and efficient, the improved sealing is experienced as soft and gentle (making the minimum needed contact of cuffs with tracheal wall), no longer causing severe reversible or permanent injury to the patient's tracheal region. The special cuff configuration comprises multiple inflatable sections (whereas the cuff being inflatable), which are possibly determined as separate balloons, and may further be provided with one or more one-directional valves, all or not being inflatable.

The present disclosure further relates to sensing and/or measuring means, particularly provided with a double cuff ET tube, for detecting if leakage occurs between the cuffs and the patient's tracheal wall. This sensing and/or measuring means may be connected with a cuff controller for controlling pressure and flow in the cuffs or here in between, in the inter-cuff region, in case leakage would occur as being detected in the inter-cuff region, which is connecting the cuffs with each other along the ventilation tube.

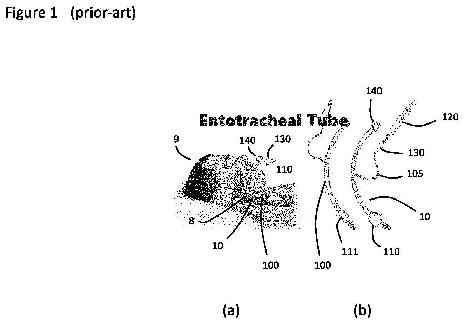

In accordance with the art, to ensure adequate mechanical ventilation, patients are intubated by using endotracheal (ET) tubes as for example illustrated in FIGS. 1A and 1B. To avoid any leakages between the tracheal wall of the patient and the ET tube, a single or double cuff is provided on the tube and being inflated to a certain low, estimated pressure. By means of example, FIGS. 1A and 1B represent a single cuff ET tube as known in the art.

In FIG. 1A, a patient 9 is depicted having an endotracheal tube 10 intubated, used to maintain the airway. The endotracheal tube 10 comprises a ventilation tube 100, having a mouth end 140 and a patient body end, this latter having a distal tip comprising of a bevel and an opening referred to as Murphy eye. At its mouth end 140 comprising a universal adapter, the tube 100 can be connected with an oxygen source. The tube 100 comprises a narrow channel 105, via which the cuff can be inflated, and hence this channel is also referred to as the cuff inflation line 105.

At a certain level, the cuff inflation line 105 comes together with the tube 100, in particular is integrated there within. The single cuff 110 is located at the distal end of the tube 100, and when inflated, it produces a seal between the trachea 8 and the cuff 110, herewith preventing aspiration and ensuring delivery of a set tidal volume when mechanical ventilation is used. An inflated cuff 110 also prevents air from passing to the vocal cords, nose, or mouth.

In FIG. 1B, the ET tube 100 is depicted with on the one hand uninflated 111 and inflated single cuff 110 respectively. In case of the ET tube 100 with inflated cuff 110, the syringe 120 for inflation is also shown. The cuff inflation line 105 comprises a pilot balloon 130, to be connected with the syringe 120 enabling inflation. The pilot balloon 130 permits air to be inserted into the cuff 110, prevents air from escaping, and can be used as a guideline for determining the presence or absence of air in the cuff 110. The universal adapter at the mouth end 140 enables attachment of the tube 100 to mechanical ventilation tubing or other types of oxygen delivery systems. When ventilation of a patient 9 has been started and e.g. suddenly leakage is detected, in order to instantly solve the problem of leakage, the applied cuff pressure may be increased by means of (further) inflating the cuff 110 using the syringe 120 being connected with the pilot balloon 130.

From experience is known that the use of conventional ET tubes as currently known in the art, may result in different issues. For example, frequent leakages may occur due to wrongly installed tubes, due to movement of the patient, or due to movement of the ET tube. Furthermore, tissue injuries may occur due to the constant or too high pressures being applied in the cuff.

The goal of the application of the new type endotracheal tube in particular having specially configured cuffs, is to ensure a leakage free ventilation by air and fluid tight sealing of the ET tube, in particular the sealing of single or double cuff with the patient's trachea wall, and herewith applying a minimum pressure in order to avoid a reversible or irreversible injury.

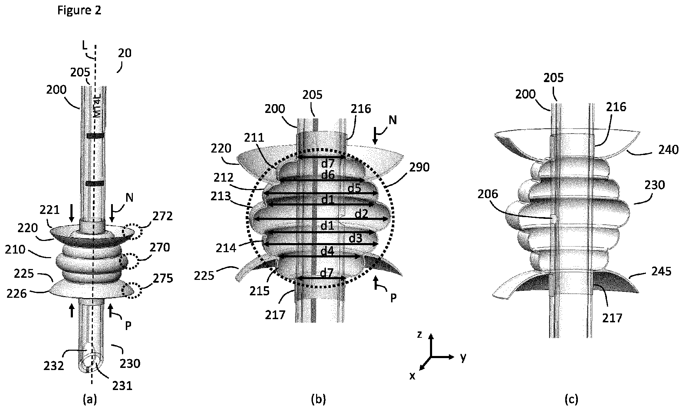

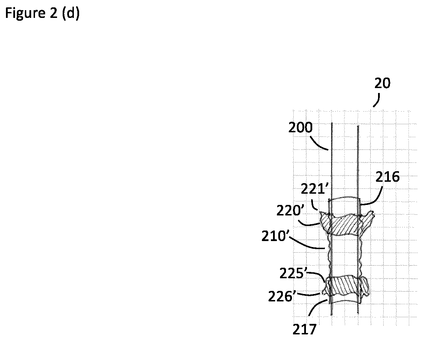

FIGS. 2A, 2B, and 2C illustrate an embodiment of a cuff configuration for a single cuff ET tube, and this in different views. A xyz coordinate system is given here for facilitating the description of the cuff configuration. Whereas these FIGS. 2A, 2B and 2C all show the inflated state, an embodiment of the single cloud cuff configuration in uninflated state is given in FIG. 2D.

In FIG. 2A the single cuff 210 is shown being provided on the ventilation tube 200 (with longitudinal axis L) of the ET tube 20 wherein the cuff inflation line 205 is marked. At the distal end 230 of the tube 200 we clearly see the bevel 231 and the Murphy eye opening 232. The cuff 210 comprises several sections 211, 212, 213, 214, 215, here by means of example five sections above each other, having the appearance of a cloud shape. Although the particular number of five sections is shown here as an example, other embodiments are also included wherein a different amount of sections (e.g. more or less than five) are foreseen. Hence, the single cuff 210 is also referred to as cloud cuff 210. Due to this configuration, we have several sections at different heights (along the z-axis) and with different or varying width or diameter (along the xy-plane). The cloud cuff is particularly designed or has a particular shape for preventing wrinkling of the cuffs (as today a common problem with ET tubes). In general, we consider a cloud cuff from the moment the cuff comprises two sections. In other words, the cloud cuff comprises at least two sections. Whereas the cuff is inflatable, the sections (comprised by the cuff) are also inflatable, and here shown in inflated view. The cloud cuff 210 comprising two or more sections is attached to the ventilation tube 200. The sections are connected, both physically and through air. At least two sections are having a (slight) difference in diameter, such that at least one section (e.g. having the largest diameter) operates as sealing portion 270, adapted to form a wrinkle-free band against a patient's tracheal wall when inflated, wherein the wrinkle-free sealing band is configured to prevent leakage of fluid or air passing the wrinkle-free sealing band when inflated.

FIG. 2B zooms in on the cuff 210 itself although in cross-section (along vertical yz-plane) and semi-transparent view such that the different parts (including at the inside) are made visible and hence can be all identified. The single cuff 210 is shown, again being provided on the ventilation tube 200 of the ET tube 20 wherein the cuff inflation line 205 is marked. The sections 211, 212, 213, 214, 215 appear as bands, lobes or ribbons on top of each other, and therefor can be referred to as band-alike. The band-alike sections have a circular shaped cross-section along the (horizontal) xy-plane. However, per band-alike section, the diameter of the circular shaped cross-section changes along the (vertical) z-axis, such that the specific band shape is generated. Such circular symmetry doesn't have to be perfect as illustrated here, but approached as close as possible, in order to follow more or less the tubular cylindrical shape of the patient's trachea when the cuff comprising (multiple) sections is inflated.

In particular, per band-alike section along the z-axis (following the z-axis in a direction upward or downward), the diameter of the section starting with a particular size (at the beginning of the section), is first increasing until a larger and maximum diameter size is reached. Further following the z-axis in the same direction (up or down), the diameter size is then decreasing again, possibly towards again the particular size as we had at the beginning of the section. Alternatively, at the end of the section, the diameter size could also be slightly smaller or larger than the particular size at the beginning of the section. By means of example, this is indicated for sections 212, 213, 214. Section 213 in particular, has a minimum diameter d1, which increases towards a maximum diameter d2, in order to generate the band-alike shape. Just below section 213, adjacent section 214 starts with the diameter d1, increasing towards diameter d3 and then decreasing again towards diameter d4. Just above section 213, adjacent section 212 starts with the diameter d1, increasing towards diameter d5 and then decreasing again towards diameter d6. Hence, the diameter per section not only changes, but also changes amongst the different sections. The cloud cuff 210 is here designed such that along the z-axis, the maximum diameter of the sections first increases, gradually in space (not in time), until maximum diameter d2 is reached, and then gradually decreases again.

A symmetric geometry is here chosen for cuff 210, such that the cuff starts and ends with the same smallest diameter d7, at respectively an opening end 216, in the form of a cylindrical or tubular section, and a closing end 217, also in the form of a cylindrical or tubular section. The diameter d7 is comparable with (or approximately the same as) the diameter of the tube 200 such that the ends 216, 217 nicely fit and close onto the surface of the tube 200. Because of the symmetry here, diameters d3, d5 and diameters d4, d6 are respectively of the same size. The cloud cuff 210 having opening end 216 and closing end 217, both being tightly fit onto the surface of the tube 200 such that their connection is sealed, and hence no air or fluid can leak there between. Here, opening and closing end 216, 217 have a diameter d7, to achieve this tight connection with the tube 200. The cloud cuff 210 with the five sections 211, 212, 213, 214, 215 as depicted here, has an overall spherical or ovoid cloud shape or volume in inflated state. Moreover, because of the multiple sections, and the symmetry in the volume, we can also recognize a (double) tapered shape in inflated state. In case for example, the cloud cuff would comprise only two sections, taking into account that the sections differ in size, i.e. one has a smaller maximum diameter than the other, the overall shape or volume would be rather conical or frustro-conical in inflated state. In case for example, the cloud cuff would comprise three sections, wherein the middle section (or band) having a larger (maximum) diameter than the outer sections (or distal and proximal bands), a double tapered shape would be represented. The cloud cuff with its different sections can be made of polyethylene terephthalate (PETP), low-density polyethylene (LDPE), polyvinyl chloride (PVC), silicone, neoprene, polyisoprene, polypropylene, or polyurethane (PU) as material. According to an embodiment, the cloud cuff material can be selected from materials such as PVC, siliconized PVC or silicone, or another physiologically compatible material. According to an embodiment, the cloud cuff material is styrene-ethylene-butylene-styrene (SEBS), or another suitable thermoplastic elastomer or polymer. According to an embodiment of the invention, the thickness of the cloud cuff is chosen very thin, being about 0,1 mm ± 25%. The cloud cuff can be made out of one piece, for example by means of a (blow) moulding process, leading to an approximately uniform thickness of for example 0,1 mm having a thickness range i.e. difference in thickness uniformity of about 5% over the entire cuff. According to an embodiment, the cloud cuff material is suitable for (blow) moulding. According to the invention, the cloud cuff material is inflatable and biocompatible.

According to the embodiment depicted here in FIGS. 2A and 2B, the cloud cuff 210 is further provided with a one-directional valve 220, 225 close to either end 216, 217 of the cuff 210, i.e. a one-directional valve 220 close to opening end (or proximal end with respect to the tube 200) and a one-directional valve 225 close to closing end 217 (or distal end with respect to the tube 200). Both valves 220, 225 are here depicted as each other's mirror image, although this is not always the case. Here, the valves 220, 225 are designed with concave surfaces. In particular, here, the concave surface of valve 220 at proximal end is directed towards the centre of the cuff, and also the concave surface of the valve 225 at the distal end is directed towards the centre of the cuff, such that the concave surfaces 220, 225 are headed to each other and hence appear as their mirror image. According to an embodiment, the valves have a different shape, and comprise for example a symmetry such that they appear the same at both proximal and distal end of the tube 200. The valves 220, 225 are for example made out of the same material as the cuff 210 and its sections 211, 212, 213, 214, 215, and the valves are possibly integral to the rest of the cuff. However, according to an embodiment, a different material for the valves could be used, and they could be provided or foreseen on the cuff as separate parts. In unused state, the valves 200, 225 appear as a flap or skirt provided onto the cloud cuff 210. The valves 220, 225 are provided for controlling high positive and negative pressure (HPNP), and are particularly configured for avoiding leakage during high ventilatory pressures or negative aspiration pressures, whereas due to such pressure, the valve gets inflated and its (concave) surface will attach to the tracheal wall to form a seal. A traditional ET tube cuff sealing will not protect against such high ventilatory pressure ventilations or during aspiration (when high negative pressures are applied).

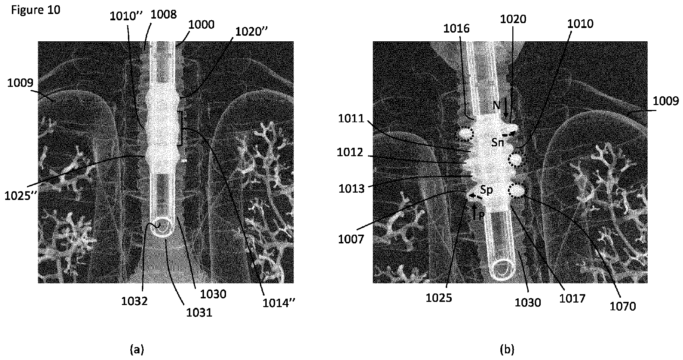

With the valves 220, 225 (or at least one of them, i.e. when having only one valve a better sealing is yet provided, whereas further improvement is achieved when having both) a solution is provided for further sealing, at low cuff pressure, even during higher ventilation pressure or during aspiration. In accordance with the state-of-the-art, positive pressure is defined as the working pressure of a ventilator used to ventilate a patient having an ET tube inserted. This positive pressure, also called overpressure, is in general maximum about 100 mbar (as legally permitted), whereas standardly, in accordance with the art, the pressure in the cuff is maximum about 20 to 30 mbar during ventilation of the patient. The pressure in the cuff, or cuff pressure, can also be higher than this 20-30 mbar but then only for a very short time, e.g. 1 to 2 minutes at most. Whereas the working pressure of the ventilator is maximum about 100 mbar, the pressure in the lungs can increase to about 100 mbar, which is considered high though possible. Negative pressure, also referred to as underpressure, is caused by lung aspiration, and hence occurs during aspiration in the lungs (or suction towards the lungs). In used or inflated state, when pressure (e.g. positive/overpressure or negative/underpressure) is applied, the valves will obtain the shape as indicated in FIGS. 2A and 2B. Due to pressure applied, the valves have opened up such that their respective edges 221, 226 make contact and hence seal with the patient's tracheal wall, at e.g. respective sealing portions 272, 275 indicated. For example, in case of positive pressure or overpressure, the working pressure of the ventilator is determining the pressure in the lungs, and due to the configuration here in FIG. 2A and 2B, of a cloud cuff 210 with valve 225, the pressure of the lungs (and directed therefrom as indicated by the arrow P) is pushing the valve 225 in particular its shell-like surface, such that it is pushed against the tracheal wall of the patient as shown in FIG. 10B. In addition to the arrow P in FIG. 10B, a dashed arrow Sp is also depicted for illustrating how the valve 1025 evolves due to the overpressure, i.e. how the surface of valve 1025 (and its inflated volume) moves towards the tracheal wall 1007 of the patient 1009. The arrows P for positive pressure are also indicated in FIG. 2A and 2B. Inspiration going along with overpressure, is maximum a few seconds per cycle. In case of for example negative pressure or underpressure, the aspiration in the lungs (and directed thereto as indicated by the arrow N) is sucking the valve 220 in particular its shell-like surface, such that it is sucked against the tracheal wall of the patient as shown in FIG. 10. In addition to the arrow N in FIG. 10B, a dashed arrow Sn is also depicted for illustrating how the valve 1020 evolves due to the underpressure, i.e. how the surface of valve 1020 (and its inflated volume) moves towards the tracheal wall 1007 of the patient 1009. The arrows N for negative pressure are also indicated in FIG. 2A and 2B. Referring back to FIG. 2A and 2B, as illustrated by their respective shape, the valves direct in opposite directions, whereas valve 220 is directed superior (meaning towards the patient's mouth), the other valve 225 is directed inferior (meaning towards the patient's lungs). More particularly, the one-directional valve will in case of (sudden and/or short but high) underpressure or overpressure make contact with the patient's tracheal wall, such that additional sealing is provided. The one-directional valve 220 will in case of underpressure make contact with the patient's tracheal wall, herewith achieving additional sealing in proximal position (towards the patient's mouth). When aspiration is occurring, leakage around the cuffs will "suck in" the air and secretions in or towards the lungs, etc. which can lead to VAP (ventilator-associated pneumonia) and other complications. The one-directional valve 220 positioned at or close to the upper or opening end 216 of the cuff 210, will create a leakage free cuff closure also during aspiration. The force or power of the aspiration will pull the valve against the tracheal wall, herewith achieving a good sealing. The one-directional valve 225 will in case of overpressure make contact with the patient's tracheal wall, herewith achieving additional sealing in distal position (towards the patient's lungs). To further improve sealing protection, and to avoid the ventilator of having major leakage during ventilation, the one-directional valve 225 is provided in the vicinity of lower or closing end 217 of the cuff 210, also referred to as distal end in view of the tube 200. This valve 225 will be subject to the pressure of the ventilator, hence creating a perfect seal. According to an embodiment of the invention, it is preferred to have at least the one-directional valve 225 provided with the cloud cuff, in order to avoid difficulties with the ventilator.

It is noted that the valves 220, 225 in FIGS. 2A and 2B are provided in between two sections, herewith achieving easier folding/unfolding of the valve as compared to the situation as depicted in FIG. 2C. In particular, in FIGS. 2A and 2B the valve 220 is provided between sections 211, 212 close to the tube cuff proximal end or opening end 216 of the cuff 210. The valve 225 is provided in between sections 214, 215 close to the tube cuff distal end or opening end 216 of the cuff 210. In general, according to an embodiment, a distal valve (meaning valve in the vicinity of the distal end) and a proximal valve (meaning valve in the vicinity of the proximal end) can be provided. More specifically, according to an embodiment, valves can be provided above and/or below sections, and/or valves can be provided between sections.

The zoom in representation on the cuff 230 in FIG. 2C shows a slightly different embodiment in that the one-directional valve 240, 245 is not provided in between two sections, but in proximity of the opening or closing end 216, 217 respectively of the cuff 230. The single cuff 230 is shown, again being provided on the ventilation tube 200 of the ET tube 20 wherein the cuff inflation line 205 is integrated. An opening 206 is illustrated here, being provided for inflating the cuff 230 via the cuff inflation line 205. Such opening (for inflation) is understood to also be present in FIGS. 2A and 2B although not shown here.

In case of standard ET tubes today, leakage may occur because of wrinkling of the cuffs. The cloud cuff in accordance with embodiments, generating a cloud shape having different sections will avoid wrinkling of the cuff, and hence leakage. As mentioned before, the section are usually small band-like, and possibly graded meaning that they appear on top of each other (when looking at FIGS. 2A-2C) from smaller to larger size and/or vice versa. As opposed to a typical cylindrical cuff (known in the art) to be inserted into a patient's trachea and inflated, the cuff outer surface is no longer a cylinder shape, but a cloud shape which can be defined as overall sphere or ovoid alike (see e.g. dotted circle/oval 290 indicated in FIG. 2B). The cuff walls defined now by different maximum diameters, are now able to inflate to their maximum (local) diameters such that they don't fold in on themselves to fit in the trachea. Hence, wrinkles or wrinkling is avoided and as a result leak paths to form are also eliminated. Current ET tubes in the art, representing leaking issues, make maintenance of adequate ventilation difficult, or even impossible. The one-directional valve in accordance with embodiments, on or close to the distal end of the cuff will avoid any leakage, even when ventilation pressure is (much) higher than the cuff pressure (wherein much is defined as 5 mbar or more). Moreover, the one-directional valve in accordance with embodiments, on or close to the proximal end of the cuff will avoid any leakage, even with (high) aspiration applied (wherein high is defined as 5 mbar or more), as opposed to standard ET tubes failing to protect against aspiration.

In FIG. 2D the single cloud cuff 210' is shown in uninflated state, being provided on the ventilation tube 200 of the ET tube 20. Due to its uninflated state, the sections are not distinguishable here, although the flap or skirt appearance of the one-directional valves 220', 225' in uninflated state are clearly visible (see shaded parts). The respective edges 221', 226' of the valves 220', 225' in uninflated state are indicated. The opening end 216 and closing end 217 of the single cuff 210' in uninflated state are depicted, both having a cylindrical or tubular shape, being tightly connected and herewith sealed to the tube 200.

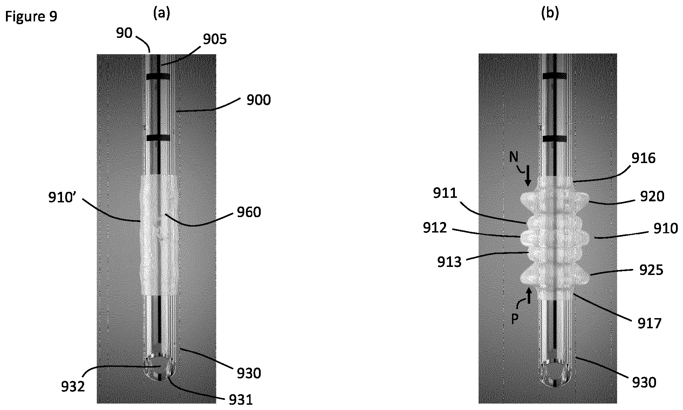

FIGS. 9A and 9B illustrates in FIG. 9A a picture embodiment of a cuff configuration for a single cuff ET tube, wherein the single cuff is shown in uninflated state, including in FIG. 9B a picture embodiment of the same single cuff configuration, now shown in fully inflated state. In FIG. 9A the single cuff 910' is shown in uninflated state, being provided on the ventilation tube 900 of the ET tube 90 wherein the cuff inflation line 905 is marked. At the distal end 930 of the tube 900 we clearly see the bevel 931 and the Murphy eye opening 932. Due to its uninflated state, the cuff appears as a flat cylindrical shape, however showing wrinkles 960 because of its volume it can take when being inflated, as illustrated in FIG. 9B. In inflated state of the cuff 910, we clearly see its sections 911, 912, 913, as well as its two one-directional (double-walled) valves 920, 925, respectively at proximal end of the cuff 910, in the vicinity of the cylindrical or tubular shaped opening end 916, and at distal end of the cuff 910, in the vicinity of the cylindrical or tubular shaped closing end 917.

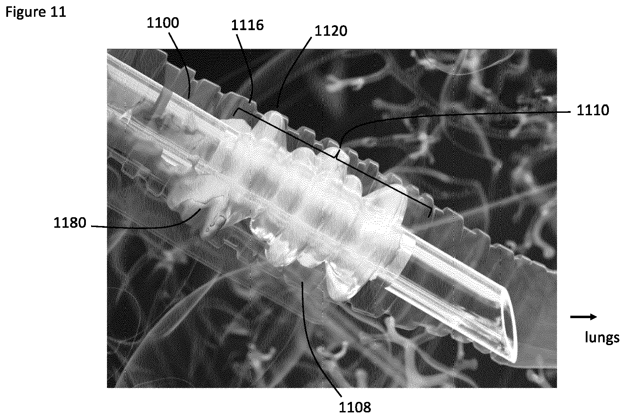

FIG. 10A and 10B illustrates in FIG. 10A a picture embodiment of a cuff configuration for a single cuff ET tube, inserted into a patient's trachea, wherein the single cuff is shown in very little inflated state, including in FIG. 10B a picture embodiment of the same single cuff ET tube in the patient's trachea, now shown with the single cuff in fully inflated state. In FIG. 10A the single cuff 1010" is shown in very little inflated (nearly uninflated) state, being provided on the ventilation tube 1000 of the ET tube, inserted into the trachea 1008 of a patient 1009. At the distal end 1030 of the tube 1000 we clearly see the bevel 1031 and the Murphy eye opening 1032. Due to its very little inflated (nearly uninflated) state, the cuff appears as an almost flat cylindrical shape. However, as opposed to the uninflated state depicted in FIG. 9A, the illustration of FIG. 10A yet shows the existence of the valves 1020", 1025" and the cuff sections 1014" starting to become visible due to very little inflation. As depicted in FIG. 10B, in fully inflated state of the cuff 1010, we clearly see its sections 1011, 1012, 1013, as well as its two one-directional (double-walled) valves 1020, 1025, respectively at proximal end of the cuff 1010, in the vicinity of the cylindrical or tubular shaped opening end 1016, and at distal end of the cuff 1010, in the vicinity of the cylindrical or tubular shaped closing end 1017. Further, in FIG. 10B, an arrow P is depicted for indicating the positive pressure or overpressure, causing the valve 1025, in particular its surface (and its inflated volume) to move towards the tracheal wall 1007 of the patient 1009, as is indicated by the dashed arrow Sp. Moreover, an arrow N is depicted for indicating the negative pressure or underpressure, causing the valve 1020, in particular its surface (and its inflated volume) to move towards the tracheal wall 1007 of the patient 1009, as is indicated by the dashed arrow Sn. With the illustration in FIG. 10B it is clearly shown that, not only the cloud cuff 1010 but also the two valves 1020, 1025 will ensure a good sealing of this cuff configuration. Examples of sealing portions are indicated in FIG. 10B by the dotted circles/ovals 1070. This good sealing, particularly at the proximal end of the cuff is further shown in FIG. 11, illustrating a picture embodiment of the same single cuff ET tube 1100 (as in FIG. 10A and 10B) in the patient's trachea 1108, shown with the single cuff 1110 in fully inflated state, and wherein secretions 1180 are blocked or prevented from moving further in the trachea 1108, specifically due to sealing by the valve 1120 at proximal end 1116, avoiding to pass the cuff configuration 1100, such that secretions 1180 cannot reach and/or enter the patient's lungs (not shown, though indicated by the arrow).

Switching to special coated ET tubes, etc. in an attempt to avoid VAP, hence paying significantly more (i.e. much higher price) per ET tube is not a complete solution. As strong leakage will not avoid VAP and the anti-bacterial coating will only be active for limited time, etc. excessive inflation of the cuff can result in tissue ischemia, ulceration, and necrosis of the tracheal wall while minor inflation may result in the leak of air and oropharyngeal secretions around the cuff which predisposes the patient to for example inadequate ventilation, de-recruitment, and aspiration pneumonia.

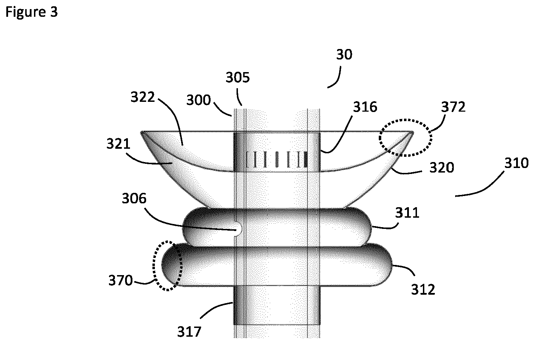

FIG. 3 illustrates another embodiment of a cuff configuration for a single cuff ET tube in accordance with the invention. The single cuff 310 is again shown being provided on the ventilation tube 300 of the ET tube 30, wherein the cuff inflation line 305 is integrated. A small opening 306 is provided in the tube 300, for inflating the cuff 310 via the cuff inflation line 305. Air comes down the line 305 and exits into the volume defined between the tube 300 and the inner walls of the cuff 310. The cloud cuff 310 comprises two sections 311, 312, above or on top of each other. The sections 311, 312 are inflatable, and here shown in inflated state. Also here, the sections are connected, either physically or through air. The two sections are having a difference in maximum diameter, such that a smaller section 311 and a larger section 312 are present. Hence, due to a difference in maximum diameter and thus folding is avoided, a sealing portion 370 is achieved, adapted to form a wrinkle-free band against a patient's tracheal wall when the cuff is inflated, wherein the wrinkle-free sealing band is configured to prevent leakage of fluid or air passing the wrinkle-free sealing band when the cuff is inflated.

The cloud cuff 310 has an opening end 316, in the form of a cylindrical or tubular section, and a closing end 317, also in the form of a cylindrical or tubular section, and both ends 316, 317 being tightly connected with the tube 300. Adjacent to the two sections 311, 312, in particular adjacent to the smaller section 311, an inflatable one-directional valve 320 is provided, which will when inflated make contact with the patient's tracheal wall, such that additional sealing is provided. Different from previous embodiment discussed with FIGS. 2A-2C, where the valve is single-walled, the one-directional valve320 in FIG. 3 is double-walled such that the valve now can also be inflated until a bowl, cup or boat-alike shape is generated. From a practical point of view, both embodiments with respectively an inflatable one-directional valve (see FIG. 3) or a singular flap or skirt (see FIGS. 2A-2C, but also FIG. 2D) are applicable for use. The double-walled valve 320 comprises an inner wall 322 and an outer wall 321. The valve 320 is in inflated state such that a sealing portion 372 (to seal against the tracheal wall) is visible. As an extension (not shown) of this embodiment, a second (mirrored) double-walled and hence inflatable one-directional valve could be provided just below the larger section 312. For completeness, it is noted that, according to an embodiment, one single-walled valve is used; or according to an embodiment, two single-walled valves are used; or according to an embodiment one double-walled valve is used; or according to an embodiment two double-walled valves are used; or according to an embodiment, a combination of a single-walled and a double-walled valve is used. In case of such combination of a single-walled and a double-walled valve used, it can be chosen where the single-walled and the double-walled valve are provided (i.e. at proximal or distal end), for example depending on the application, need, anatomy of the patient and/or other circumstances.

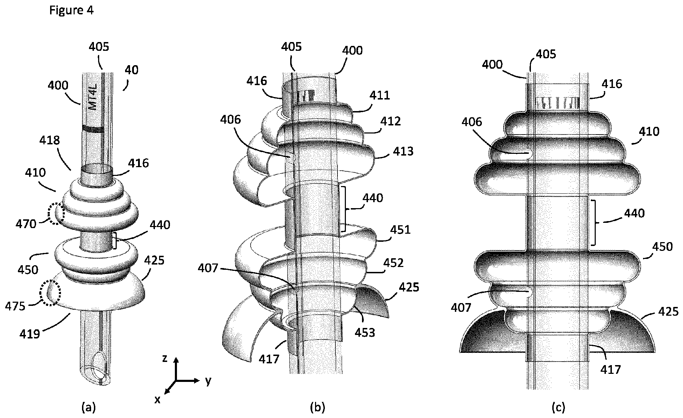

FIGS. 4A, 4B, and 4C illustrate an embodiment of a cuff configuration for a double cuff ET tube.

As shown in FIG. 4A, the double cuff ET tube 40 comprises two cuffs 410, 450 provided on the ventilation tube 400 of the ET tube 40, wherein the cuff inflation line 405 is marked. The two cuffs 410, 450 are both cloud cuffs, each comprising here three sections (by means of example, i.e. less or more sections are also understood under the invention, even asymmetric cases are not excluded, wherein one cuff has more sections than the other). According to an embodiment, the double cuff configuration is made as one part (e.g. by means of a (blow) moulding process), wherein the two cuffs 410, 450 are connected by a cylindrical or tubular section. Hence, the cuffs 410, 450 are at a fixed distance from each other. The space in between the two cloud cuffs 410, 450, can be referred to as inter-cuff region 440 where the cylindrical or tubular connection is provided, connecting the upper cloud cuff 410 with the lower cloud cuff 450 (otherwise being separated). According to the invention, a double cloud cuff has no significantly different impact compared to a single cloud cuff regarding sealing aspect. However, the double cuff configuration with its inter-cuff region has an advantage from a measurement point of view. The inter-cuff region has the functional advantage of facilitating pressure or flow-related measurements in this particular region, while using a measurement device such as e.g. further referred to as a cuff controller (not only measuring but also controlling). At the double cuff proximal end 418, we have the opening end 416, in the form of another cylindrical or tubular section, adjacent to the upper cloud cuff 410, and being tightly connected with the tube 400. FIG. 4B in perspective, and FIG. 4C in plane, zoom in on the cuffs 410, 450 although in cross-section (along vertical yz-plane) and semi-transparent view.

In FIG. 4B is indicated that the upper cloud cuff 410 comprises three sections 411, 412, 413, one above the other, and here shown in inflated state. The lower cloud cuff 450 at distal end 419 comprises three sections 451, 452, 453, on top of each other, while also shown here in inflated state. The three sections per cuff are having a difference in maximum diameter, such that a smaller section 411, 453, a middle section 412, 452 and a larger section 413, 451 for the respective cuffs 410, 450 are present. Both cloud cuffs 410, 450 are depicted as each other's mirror image, while having each a frustro-conical shape. Per cuff, a sealing portion 470 (only indicated for the upper cuff 410) is achieved, adapted to form a wrinkle-free band against a patient's tracheal wall when the cuff is inflated, wherein the wrinkle-free sealing band is configured to prevent leakage of fluid or air passing the wrinkle-free sealing band when inflated.

As depicted in FIGS. 4B and 4C, at the double cuff distal end 419, we have the closing end 417, in the form of further cylindrical or tubular section, adjacent to the lower cloud cuff 450, and being tightly connected with the tube 400. An opening 406, 407 is provided, for inflating the cuffs 410, 450 via the cuff inflation line 405. For cuff 410, the sections 411, 412, 413 are connected, physically and through air. For cuff 450, the sections 451, 452, 453 are connected, physically and through air.

According to the embodiment depicted here in FIGS. 4A-4C, the cloud cuff 450 is provided with one-directional valve 425 close to the closing end 417. In unused state, the valve 425 appears as a flap or skirt provided onto the cloud cuff 450, in particular provided in between two sections 452, 453 for easy folding/unfolding of the valve 425. The valve 425 is in inflated state such that a sealing portion 475 (to seal against the tracheal wall) is visible. Both cloud cuffs 410, 450, including each of their three sections, as well as the one-directional valve 425 can be made of the same material, such as for example polyethylene terephthalate (PETP), low-density polyethylene (LDPE), polyvinyl chloride (PVC), silicone, neoprene, polyisoprene, polypropylene, or polyurethane (PU). Whereas, in practice, a single cloud cuff can be provided as standalone, to replace a standard cuff on an ET tube, a double cloud cuff is general provided as integrated together with an ET tube, wherein a cuff controller is also to be considered, as connected or connectable for measuring and control purposes.

Also in the configuration of double cuff ET tube, comprising cloud cuffs, forming folds of the cuff along its contact with the trachea wall, is no longer a concern.

Recent cuffs are made of very thin material and can overcome some of the constraints of previous-generation cuffs. A very thin cuff wall forms smaller folds and thus allows less leakage. Mucus leakage past the cuff is a clinically important risk that could be prevented with a very thin cuff made of a highly distensible elastomer.

The cloud cuffs, comprising multiple sections at different heights and with varying maximum diameter (due to their configuration), wherein the sections being connected together, are configured to avoid folding and thus potential leakage of the ET tube cuffs and to guarantee a good sealing and tight fit.

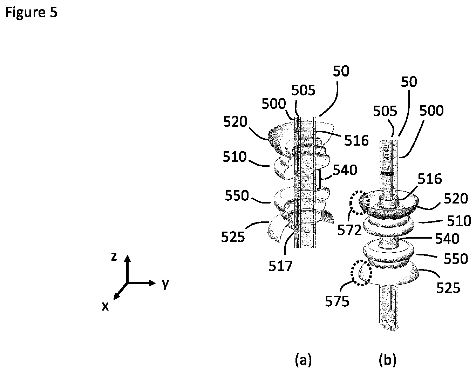

FIGS. 5A and 5B illustrate another embodiment of a cuff configuration for a double cuff ET tube. Two one-directional valves are provided with the double cuff configuration. FIG. 5A depicts cross-section (along vertical yz-plane) and FIG. 5B depicts full perspective zoom in on the cuffs 510, 550. The double cuff ET tube comprises two cuffs 510, 550 is provided on the ventilation tube 500 of the ET tube 50, wherein the cuff inflation line 505 is marked. The two cuffs 510, 550 are both cloud cuffs, each comprising three sections.

In between the two cloud cuffs 510, 550, an inter-cuff region 540 is indicated, connecting the upper cloud cuff 510 with the lower cloud cuff 550. At the double cuff proximal end, the opening end 516, in the form of a cylindrical or tubular section, is adjacent to the upper cloud cuff 510, and being tightly connected with the tube 500. At the double cuff distal end, the closing end 517, also in the form of a cylindrical or tubular section, is adjacent to the lower cloud cuff 550, and being tightly joint or connected with the tube 500. Both cloud cuffs 510, 550 comprise a one-directional valve 520, 525 close to their respective ends 516, 517. In unused state, both valves 520, 525 appear as a flap or skirt provided onto their corresponding cloud cuff 510, 550, in particular provided in between two sections for easy folding/unfolding of the valve. Again here, the valves will in case of (sudden and/or short but high) underpressure or overpressure make contact with the patient's tracheal wall, such that additional sealing (see e.g. sealing portions 572, 575) being provided. Hence, the embodiment is offering a good protection (against leakage) even during aspiration and higher pressure ventilation.

As known from the art, leakage may be caused by higher ventilation pressures given by the ventilator, or by high aspiration. When the cuffs from the art are e.g. filled with/at 25 mbar, but the ventilation pressure is 40 mbar, then the cuffs will not have sufficient sealing, hence leakage and limitation of the ventilator capacity.

Referring back to the embodiment of FIGS. 5A and 5B, the cloud cuffs include multiple sections at different heights and having varying maximum diameter (due to their configuration), wherein the sections being connected together, in order to avoid folding and thus potential leakage of the ET tube cuffs and to guarantee a good sealing and tight fit. Additionally, a flap or valve 525 is provided at the distal end of the ET tube double cuff, which will close itself (against the tracheal wall) with the ventilator pressures. Pressure of the ventilator will open the valve and create the tight fitting, avoiding the cuff to leak. Hence, a cuff with lower pressure than the ventilator pressure, will still ensure sealing. In any case, the cuff is at a certain pressure, referred to as cuff pressure, which will be different in inflated versus uninflated state. In uninflated state, the cuff pressure is 0 mbar (or vacuum). In inflated state, standardly, the cuff pressure is at 20-30 mbar, although it may peak also to 60-70 mbar for maximum a few minutes, i.e. about 1 to 2 minutes. The working pressure of the ventilator, and hence (comparable with) the lung pressure, is in general significantly higher, e.g. 50 mbar, up to 100 mbar. In case of leakage, it is possible that the ventilator gives an alarm signal, and even possibly the ventilator may go in failure due to leakage at or on the cuff. With the one-directional valve provided at the distal end of the ET tube double cuff, such as valve 525 in FIG. 5, but also as valve 225 in FIG. 2 and valve 425 in FIG. 4, leakage can be avoided, and hence all possible effects thereof, such as for example a ventilator turning into failure state. Referring back to the embodiment of FIGS. 5A and 5B, for aspiration, a flap or valve 520 is provided at the proximal end of the ET tube double cuff.

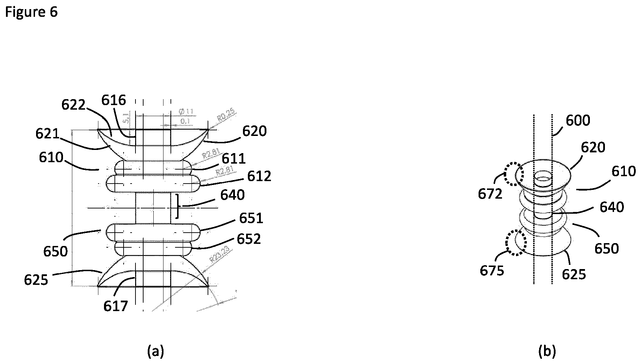

FIGS. 6A and 6B illustrate yet another embodiment of a cuff configuration for a double cuff ET tube. The embodiment can be interpreted as the double cuff version of the single cuff configuration being illustrated in FIG. 3, whereas now the cloud cuff comprising two sections and an inflatable one-directional valve is now duplicated though mirrored (i.e. one is the mirror image of the other in the configuration) in order to create a double cuff configuration.

FIG. 6A depicts a cross-section (along vertical yz-plane), and FIG. 6B depicts a full perspective zoom in on the cuffs 610, 650. The cloud cuffs 610, 650 comprise each two sections 611, 612, 651, 652 on top of each other, and here shown in inflated state. Per cuff, the sections are connected, and are having a difference in maximum diameter, such that a smaller section 611, 652 and a larger section 612, 651 are present. Hence a sealing portion is achieved, adapted to form a wrinkle-free band against a patient's tracheal wall when inflated, wherein the wrinkle-free sealing band is configured to prevent leakage of fluid or air passing the wrinkle-free sealing band when inflated. The cloud cuff 610 is adjacent to opening end 616, while the cloud cuff 650 being adjacent to closing end 617, for tightly connecting with the tube. Both opening end 616 and closing end 617 are in the form of a cylindrical or tubular section. Adjacent to the smaller section 611 of cloud cuff 610, an inflatable one-directional valve 620 is provided, whereas adjacent to the smaller section 652 of cloud cuff 650, an inflatable (mirrored) one-directional valve 625 being provided. Both valves 620, 625 will when the cuff is inflated make contact with the patient's tracheal wall, such that additional sealing is provided. The one-directional valves being inflatable means that they are now double-walled such that they can be inflated until a bowl, cup or boat-alike shape is generated. From a practical point of view, both inflatable one-directional valves as well as singular flaps or skirts can be used. As indicated in FIG. 6A, the double-walled valve 620, comprises an inner wall 622 and an outer wall 621. As indicated in FIG. 6B, the valves 620, 625 are in inflated state such that respective sealing portions 672, 675 (to seal against the tracheal wall) are visible. The dotted lines in FIG. 6B, indicate the positioning of the ventilation tube 600, onto which the cuff configuration is to be provided.

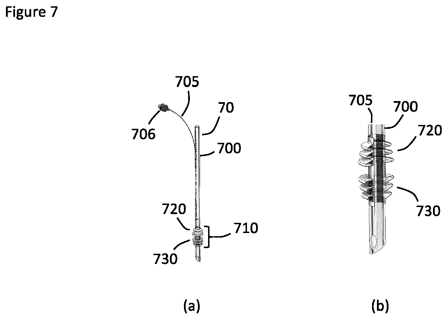

FIGS. 7A and 7B illustrate a further embodiment of a cuff configuration 710 for a double cuff ET tube 70. The embodiment is different from the one being illustrated in FIGS. 4A-4C, in that now no one-directional valve is provided with the double cuff configuration 710. While in FIG. 7A the entire double cuff ET tube 70 is depicted with ventilation tube 700, cuff inflation lumen or line 705 (provided with pilot balloon 706) and two cloud cuffs 720, 730, FIG. 7B zooms in on the two cloud cuffs attached around the tube. It is noted that the ribbed shapes of the cuffs 720, 730 here differ from the rounded shapes in FIGS. 4A-4C. Although the rounded shapes being preferred, ribbed shaped are not necessarily excluded from the invention.

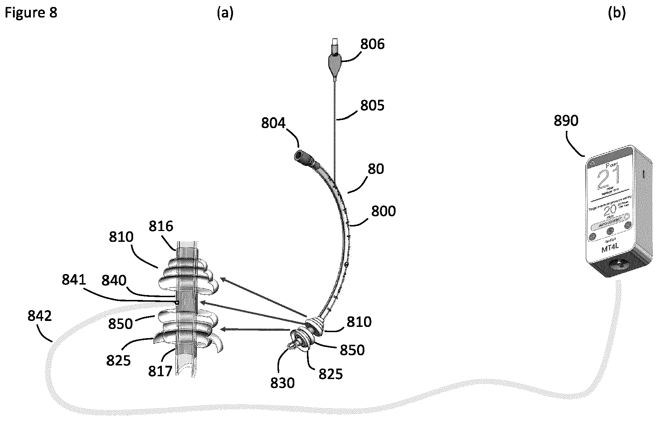

According to an aspect of the invention, in addition to the single or double cloud cuff, and the one or more one-directional valves to be provided with single or double (standard or cloud) cuffs, a cuff controller is also provided. Before describing embodiments as illustrated in the attached drawings of FIG. 8A and 8B regarding the cuff controller, in accordance with the invention, a more general description on the cuff controller is first given. With a controller (like the cuff controller) is meant an (electronic) device generating control signals for other instruments (by executing a control algorithm based on monitoring or measuring or sensing one or more signals). In the context of the invention, such device or instrument is any device suited for providing a flow of air (such as a ventilator) and hence when providing such flow to a closed or semi-closed environment (like a cuff or an inter-cuff region) also determines the pressure. The (electronic) device comprises a storage device (e.g. to store user defined setpoints) (like memory) and a computation device (like a microcontroller or microprocessor) generating control signals and being able to execute measurement and control procedures or protocols.

In a first embodiment, a cuff controller is provided that automatically controls the pressure inside the cuffs of an endotracheal tube (ETT). Such cuff controller inputs measurements of such pressure and generates control signals (as discussed above) based on such measurements.

In a second embodiment, a cuff controller is provided, particularly when using an ETT having two cuffs, wherein the pressure of the interspace between the cuffs (i.e. inter-cuff region) can be measured. The cuff controller can now also provide detection of leakage around the cuffs (instead of therein). The cuff controller can automatically increase the pressure inside the cuffs, in case it detects a leakage, which can be the result of inappropriate sealing due to e.g. too little inflation of the cuffs and/or wrinkles in the cuff surface. In addition, the cuff controller can suck secretions that would build up above the cuffs. Such cuff controller inputs measurements of pressure with cuff and/or pressure in the interspace between the cuffs (i.e. inter-cuff region) and generates control signals (as discussed above) based on such measurements. Moreover it can generate control signals for a pump to suck secretions.

In a further advanced embodiment of this second embodiment, the cuff controller can as part of a measurement procedure or protocol, temporarily decrease the pressure in the inter-cuff region and then measure or monitor the pressure evolution, to check if the pressure changes, because any change of pressure indicates a leakage. It is noted that first and second embodiment above, can be combined.

According to an embodiment of the invention, the cuff controller has multiple functionalities. These can be for example (but not limited thereto), in random order of appearance: (1) regulate the pressure inside the cuffs to a user defined setpoint between 10 mbar and 50 mbar, (2) detect cuff leakage because of damaged cuff, (3) detect tracheal leakage around the cuffs, (4) have an automatic cuff pressure adjustment mode, i.e. automatically increase the pressure inside the cuffs within a user defined tolerance range between 5 mbar and 20 mbar to provide appropriate sealing when a tracheal leakage is detected, (5) have a time limited hold mode, i.e. the cuff pressure is temporarily increased to a certain higher target pressure to provide a better sealing during critical situations (e.g. vomiting, repositioning patient or ETT, suctioning, etc.); in case the double cuff ETT is used, the negative pressure in the interspace is also increased to keep the flaps of the cuffs closed (even when suctioning is performed), (6) suction of secretions above the cuff, (7) remain functional without mains supply for some time (battery), and (8) give audible and visual alarms (cuff leakage, tracheal leakage, battery, etc.)

According to an embodiment of the invention, the cuff controller has different operating modes. When starting up the cuff controller, the user must indicate what kind of ETT is used: a standard ETT with a single cuff, or an ETT with double cuff. For the ETT with double cuff, for example following five modes exist on the cuff controller.
1. Controlled cuff pressure mode. Most basic mode (also present in competitor devices) where the cuffs are inflated to a certain pressure and this pressure is maintained over time. Settings: Target pressure.
2. Controlled cuff pressure mode with tracheal leak detection. Same as previous mode but while the cuffs are at the target pressure a negative pressure is created in the interspace between the cuffs to detect tracheal leaks. If a leak is detected (too fast of a pressure rise in interspace) the user is notified by an audible alarm. Settings: Target pressure.
3. Automatic cuff pressure adjustment mode. Same as previous mode but when a tracheal leak is detected, the pressure inside the cuffs is automatically increased by a certain amount. The user defines a minimum and a maximum target pressure and this mode tries to find the minimal pressure that provides a leak free tracheal seal by starting at the minimum pressure and increasing the pressure stepwise until there is no leakage anymore. If the maximum target pressure is reached and a leak is still present, the user is notified by an audible alarm. Settings: Minimum target pressure. Maximum target pressure.
4. Time limited hold mode. In this mode the cuff pressure is temporarily increased to a certain higher target pressure to provide a better seal during critical situations (e.g. vomiting, repositioning patient or ETT, suctioning, etc.). In case the double cuff ETT is used, the negative pressure in the interspace is also increased to keep the flaps of the cuffs closed (even when suctioning is performed). Because increased cuff pressures can cause tissue necrosis this function is time limited. After the user defined time has elapsed, the controller automatically returns to the mode it was in at the settings it was in. Settings: Augmented target pressure. Hold time.
5. Deflate mode. In this mode the pressure inside the cuffs is lowered to zero so the patent can be safely extubated. No settings

When using a single cuff ETT, for example only following three modes exist, all with the same properties as described above: 1/ Controlled cuff pressure mode, 2/ Time limited hold mode, and 3/ Deflate mode.

According to an embodiment of the invention, the cuff controller has following five functional requirements.
1. The cuff controller must regulate the pressure inside the cuffs to a user defined setpoint between 10 mbar and 50 mbar. Cuffs are automatically inflated to the set pressure and this pressure is maintained over time to provide an adequate seal to prevent backward leakage of tidal volume (risk of hypoxia and/or anaesthetic agent pollution of the operating theatre) or forward leakage of fluids into the lungs (risk of aspiration and/or pneumonia). Reason: lower pressure (underinflation) would not close off the trachea and higher pressures (overinflation) can cause tissue necrosis in the tracheal wall by blocking mucosal blood flow. Automatic pressure monitoring and control unburdens healthcare professionals of doing this manually. This is a basic feature inherent to a cuff
2. The cuff controller must be able to detect cuff leakage (damaged cuff) in all operating modes and notify the user with an audible and visual alarm. A leakage because of a damaged cuff could for example be detected when the pressure inside the cuffs cannot be maintained without continuous pumping air into them. The alarm notifies the healthcare professionals of the leakage so they can intervene to avoid the risks associated with inappropriate cuff seal as described earlier. Reason: this is a basic safety feature also present in all cuff controllers in the art.
3. When tracheal leak detection mode is active the cuff controller must detect leakage around the cuffs. The leakage around the cuffs (tracheal leakage) could for example be detected by depressurizing the interspace between the cuffs and checking for a pressure rise. Reason: this is a unique feature that does not exists on the market yet and decreases the risks associated with inappropriate seal of the cuffs and unburdens healthcare professionals.
4. When automatic cuff pressure adjustment mode is active and a tracheal leak is detected the cuff controller must automatically increase the pressure inside the cuffs within a user defined tolerance range between 5 mbar and 20 mbar to provide appropriate seal. Description: this mode assures good tracheal seal without burdening healthcare professionals. Reason: this is a unique feature that does not exists on the market yet and decreases the risks associated with inappropriate seal of the cuffs and unburdens healthcare professionals.
5. When automatic cuff pressure adjustment mode is active the cuff controller must give an audible and visual alarm in case the leakage cannot automatically be resolved by augmenting the pressure inside the cuffs within the user set tolerance range. The alarm notifies the healthcare professionals of the leakage so they can intervene to avoid the risks associated with inappropriate cuff seal as described earlier. Reason: avoid the risks associated with inappropriate cuff seal as described earlier.

FIGS. 8A and 8B illustrates in FIG. 8A an embodiment of a type of double cuff ET tube comprising a double cuff configuration, wherein the tube is provided with sensing and/or measuring system, for detecting if leakage occurs between the cuffs and the patient's tracheal wall, including in FIG. 8B a cuff controller to be connected therewith. As shown in FIG. 8A, the double cuff ET tube 80 comprises two cuffs 810, 850 provided on the ventilation tube 800 of the ET tube 80, wherein the cuff inflation line 805 is marked and provided with a pilot balloon 806. The mouth end 804 and distal end 830 of the ET tube are also indicated. The two cuffs 410, 450 are both cloud cuffs, each comprising here three sections. In between the cuffs 810, 850, also called the inter-cuff region 840, the cuffs are connected by means of a cylindrical or tubular section. At the double cuff proximal end, we have the opening end 816, in the form of another cylindrical or tubular section, adjacent to the upper cloud cuff 810, and being tightly connected with the tube 400. At the double cuff distal end, we have the closing end 817, in the form of a further cylindrical or tubular section, adjacent to the lower cloud cuff 850, and being tightly connected with the tube 400. The lower cloud cuff 850 is provided with one-directional valve 825 in the vicinity of the closing end 817. The cylindrical or tubular section located at the inter-cuff region 840 is for example provided with a small opening 841, for connecting a thin tube 842 therewith, which thin tube 842 is also connected with the cuff controller 890 depicted in FIG. 8B. Via this thin tube connection, the pressure in the inter-cuff region 840 can be measured and displayed by the cuff controller 890. The type of double cuff ET tube 80 of FIG. 8A is in fact comparable to the one as shown in FIGS. 4A-4C, although now also referring to allowing measurement in between the cuffs 810, 850, i.e. in the inter-cuff region 840, in order to detect leakage and to actively correct pressures. In other words, the possibility is now being proposed to measure the pressure around the cuff and to detect any possible leakage between the cuffs and the tracheal wall. Via the small opening 841 and thin tube connection 842, the space between the cuffs or inter-cuff region can also be connected to a pressure inlet (e.g. of a ventilator), such that pressure can be added and/or adjusted, and herewith a balanced pressure in and between the cuffs is being generated. Standard ET tubes may allow measurement of the cuff pressure, however this does not necessarily reveal the leakage related to the cuff-trachea closure. By measuring around i.e. in between the cuffs, a leakage can be detected and corrected for. For example, first measure the pressure in the inter-cuff region by means of connecting the small opening and thin tube with the cuff controller. Connect the thin tube also with a pump, such that air can be sucked (e.g. for about 20 mbar) from the inter-cuff-region and hence a small vacuum is created. Then, the evolution of pressure in the inter-cuff region is monitored with the cuff controller. In case the pressure changes, one can conclude that there is a leakage. Having the thin tube then connected with the pump or pressure inlet, the pressure can be adjusted and corrected for.

In accordance with an embodiment, the double cuff ET tube with cloud cuffs is provided in between the cloud cuffs with sensing and/or measuring means, to be connected with a cuff controller. This cuff controller is an electronic medical device, that can measure and control by means of a feedback mechanism the cuff pressure and leakage detection of the air and fluid tight seal. Hence, the cuff controller not only can allow to detect a leakage around the cuffs, but may also allow for active automatic correction.

The cuff controller measures and sustains the cuff pressure, but also detects whether the cuffs are giving the necessary protection and seal (by measuring the pressure between the two cuffs, leakage on the fitting is immediately detected). Therefore, it will automatically adapt to the needed target pressures (within the set tolerance) to maintain a leakage free cuffed ET tube. Hence, an actively measuring and pressure/flow corrective device, offering optimal leakage free intubation.

With the cuff controller, a user preset pressure is given to start filling the cuffs. By means of a feedback mechanism, when the pressure between the two cuffs is decreasing, or a higher flow in or out is needed to keep the pressure constant at a higher or a lower value between both cuffs, hence not having an airtight fit sealed cuff, the cuff controller will increase the cuff pressure till the flow decreases to keep the pressure constant, ensuring a perfect fit at the lowest possible pressure in the cuffs.

This feature makes the device unique as a guaranteed air tightly fit seal with the lowest possible cuff pressure is applied, to avoid tissue necrosis, etc. Currently, it is difficult for the user to know what pressure to put on the cuffs to have occlusion. That is why cuffs are usually inflated at 20 to 30 cm H₂O or mbar, i.e. a predetermined and patient-safe allowable pressure, and temporarily (for maximum a few minutes) sometimes even a lot higher (e.g. up to 60 or 70 mbar which is still allowable if only for a short i.e. 1 or 2 minutes time). Since we measure whether there is a leak (or not), we also know what pressure (e.g. because the evolution of the pressure is monitored) is needed to be leak-free. In other words, it is possible that only 15 cm H₂O or mbar pressure is needed for closure or sealing, while today a minimum of 20 cm H₂O or mbar is already taken as standard. The devices described herein may avoid also unintentional leakage, which in turn avoids other complications such as VAP, etc.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue. The term "substantially" is used herein also to represent the degree by which a quantitative representation may vary from a stated reference without resulting in a change in the basic function of the subject matter at issue. Thus, it is used to represent the inherent degree of uncertainty that may be attributed to any quantitative comparison, value, measurement, or other representation, referring to an arrangement of elements or features that, while in theory would be expected to exhibit exact correspondence or behavior, may in practice embody something less than exact.

Unless otherwise defined, all technical and scientific terms used herein have the same meaning as commonly understood by one of ordinary skill in the art to which the invention belongs. The terminology used in the description herein is for describing particular embodiments only and is not intended to be limiting. As used in the specification and appended claims, the singular forms "a," "an," and "the" are intended to include the plural forms as well, unless the context clearly indicates otherwise.

As used herein, the terms "horizontal" and "vertical" are relative terms only, are indicative of a general relative orientation only, and do not necessarily indicate perpendicularity. These terms also may be used for convenience to refer to orientations used in the figures, which orientations are used as a matter of convention only and are not intended as characteristic of the devices shown. The present invention and the embodiments thereof to be described herein may be used in any desired orientation. Moreover, horizontal and vertical walls need generally only be intersecting walls, and need not be perpendicular.

It is noted that one or more of the following claims utilize the term "wherein" as a transitional phrase. For the purposes of defining the present technology, it is noted that this term is introduced in the claims as an open-ended transitional phrase that is used to introduce a recitation of a series of characteristics of the structure and should be interpreted in like manner as the more commonly used open-ended preamble term "comprising."

It should be understood that where a first component is described as "comprising" or "including" a second component, it is contemplated that, in some embodiments, the first component "consists" or "consists essentially of" the second component. Additionally, the term "consisting essentially of" is used in this disclosure to refer to quantitative values that do not materially affect the basic and novel characteristic(s) of the disclosure.

It should be understood that any two quantitative values assigned to a property or measurement may constitute a range of that property or measurement, and all combinations of ranges formed from all stated quantitative values of a given property or measurement are contemplated in this disclosure.

While particular embodiments have been illustrated and described herein, it should be understood that various other changes and modifications may be made without departing from scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

## Claims

1. A cloud cuff (210; 910) for an endotracheal tube (ETT) system, the cloud cuff being attachable to a ventilation tube of the ETT system, and the cloud cuff comprising: (i) a first cylindrical part provided at a proximal end (216; 916) of the cloud cuff and a second cylindrical part provided at a distal end (217; 917) of the cloud cuff, wherein the first and second cylindrical parts have a surface matching a cylindrical shape of the ventilation tube (200; 900); (ii) a proximal one-directional valve (220; 920) at the proximal end of the cloud cuff, the proximal one-directional valve being inflatable; (iii) a distal one-directional valve (225; 925) at the distal end of the cloud cuff, the distal one-directional valve being inflatable; and (iv) an inflatable cuff body (290) between the proximal end (216; 916) and the distal end (217; 917);
wherein:
- the inflatable cuff body (290) having a cloud shape with at least two sections having a difference in maximum diameter, the sections (211-215; 911-913) having a gradually changing diameter along the length of the ventilation tube (200; 900), said sections comprising rounded shapes having convex surfaces, wherein
∘ at least one section (213; 912) operates as sealing portion (270) adapted to form a wrinkle-free band against a patient's tracheal wall when inflated, and having a maximum diameter (d2); and
∘ the at least two sections (212, 213; 911, 912), having a difference in maximum diameter (d3, d2), comprise said at least one section (213; 912) and a smaller section (212; 911) having a smaller maximum diameter (d3) than the maximum diameter (d2) of the at least one section (213; 912) and wherein said smaller section (212; 911) is not touching the patient's tracheal wall;
- the proximal one-directional valve (220; 920) in case of a negative pressure defined as underpressure making contact with the patient's tracheal wall, and providing herewith additional sealing portion (272) with the patient's tracheal wall in proximal position; and
- the distal one-directional valve (225; 925) in case of a positive pressure defined as overpressure making contact with the patient's tracheal wall, and providing herewith further additional sealing portion (275) with the patient's tracheal wall in distal position.

2. The cloud cuff (210; 910) of claim 1, wherein:
the proximal one-directional valve (220; 920) comprises a concave surface at the proximal end directed towards a centre of the cloud cuff, and
the distal one-directional valve (225; 925) comprises another concave surface at the distal end directed towards the centre of the cloud cuff,
such that the concave surfaces are headed to each other and hence appear as their mirror image.

3. The cloud cuff (210; 910) of claim 1 or 2, wherein the proximal one-directional valve and the distal one-directional valve is double-walled.

4. The cloud cuff (210; 910) of claim 1 to 3, having an approximately uniform thickness, in particular the thickness is achieved by means of (blow)moulding the cloud cuff during manufacturing thereof, and is in the range of 0,05 mm and 0,2mm, with a uniformity in the range of ±5%.

5. The cloud cuff (210; 910) of claims 1 to 4, being configured, by selection of the to be used materials and/or its thickness, to be inflated to cuff pressures from 5 cm H₂O to 30 cm H₂O or cuff pressures of up to 100 cm H₂O.

6. The cloud cuff (210; 910) of claims 1 to 5, being made from polyethylene terephthalate (PETP), low-density polyethylene (LDPE), polyvinyl chloride (PVC), silicone, neoprene, polyisoprene, polypropylene, or polyurethane (PU).

7. The cloud cuff (210) of claims 1 to 6, wherein the proximal one-directional valve (220) is provided in between two sections (211, 212).

8. The cloud cuff (210) of claims 1 to 7, wherein the distal one-directional valve (225) is provided in between two sections (214, 215).

9. The cloud cuff (210; 910) of claims 1 to 8, wherein the sections (211-215; 911-913) form an overall spherical or ovoid cloud shape or volume in inflated state, or else the overall shape or volume is conical or frustro-conical in inflated state.

10. The cloud cuff (210; 910) of claims 1 to 9, wherein:
the maximum diameter (d3, d5) of the sections (211, 212, 214, 215; 911, 913) first increases, gradually in space, until the maximum diameter (d2) of the at least one section (213; 912) is reached, and then gradually decreases again.

11. The cloud cuff (210) of claim 10, wherein:
the proximal one-directional valve (220) is provided onto the inflatable cuff body (290) in the vicinity of the proximal end (216), in between two sections (211, 212); and
the distal one-directional valve (225) is provided onto the inflatable cuff body (290) in the vicinity of the distal end (217), in between two sections (214, 215).

12. An endotracheal tube (ETT) (20, 40) system for ventilating a patient, the ETT system comprising:
- a cloud cuff (210, 410) according to claims 1 to 11;
- one or more cuff inflation lumens; and
- a ventilation tube (200, 400).

13. The ETT (20, 40) system of claim 12, wherein the ventilation tube comprising an opening (206, 406) for inflating the cloud cuff via a cuff inflation lumen (205, 405) integrated in the ventilation tube.

14. The ETT (40) system of claim 12 or 13, wherein the cloud cuff (410) is defined as primary cloud cuff, the ETT system further comprising:
- an additional cloud cuff (450) according to claims 1 to 11, defined as secondary cloud cuff in a distal position with respect to primary cloud cuff, wherein the two cloud cuffs (410, 450) being provided with one or more cuff inflation lumens to inflate and/or deflate the two cuffs;
- a means for sensing and/or measuring airflow parameters in an inter-cuff region connecting primary cloud cuff with secondary cloud cuff; and
- a cuff controller comprising (i) an electronic device for inputting sensed and/or measured airflow parameters, and computing one or more regulated flows of air from the airflow parameters, and (ii) one or more first mechanical means for providing the regulated flows of air.

15. The ETT (40) system of claim 14, further comprising a non-transitory machine-readable storage medium storing a computer program product, operable on a processing engine, for executing said computing of the electronic device.

## Patentansprüche

1. Wolkenmanschette (210; 910) für ein Endotrachealtubussystem (ETT-System), wobei die Wolkenmanschette an einen Beatmungstubus des ETT-Systems anbringbar ist, und die Wolkenmanschette umfassend: (i) einen ersten zylindrischen Teil, der an einem proximalen Ende (216; 916) der Wolkenmanschette bereitgestellt ist, und einen zweiten zylindrischen Teil, der an einem distalen Ende (217; 917) der Wolkenmanschette bereitgestellt ist, wobei der erste und der zweite zylindrische Teil eine Oberfläche, die mit einer zylindrischen Form des Beatmungstubus (200; 900) übereinstimmt, aufweisen; (ii) ein proximales Einwegventil (220; 920) an dem proximalen Ende der Wolkenmanschette, wobei das proximale Einwegventil aufblasbar ist; (iii) ein distales Einwegventil (225; 925) an dem distalen Ende der Wolkenmanschette, wobei das distale Einwegventil aufblasbar ist; und (iv) einen aufblasbaren Manschettenkörper (290) zwischen dem proximalen Ende (216; 916) und dem distalen Ende (217; 917);
wobei:
- der aufblasbare Manschettenkörper (290) eine Wolkenform mit mindestens zwei Bereichen aufweist, die eine Differenz in maximalem Durchmesser aufweisen, wobei die Bereiche (211-215; 911-913) einen sich allmählich ändernden Durchmesser entlang der Länge des Beatmungstubus (200; 900) aufweisen, die Bereiche umfassend abgerundete Formen, die konvexe Oberflächen aufweisen, wobei
∘ mindestens ein Bereich (213; 912) als Dichtungsabschnitt (270) fungiert, der angepasst ist, um, wenn er aufgeblasen ist, ein faltenfreies Band an einer Trachealwand eines Patienten auszubilden, und er einen maximalen Durchmesser (d2) aufweist; und
∘ die mindestens zwei Bereiche (212, 213; 911, 912), die eine Differenz in maximalem Durchmesser (d3, d2) aufweisen, den mindestens einen Bereich (213; 912) und einen kleineren Bereich (212; 911), der einen kleineren maximalen Durchmesser (d3) als den maximalen Durchmesser (d2) des mindestens einen Bereichs (213; 912) aufweist, umfassen, und wobei der kleinere Bereich (212; 911) die Trachealwand des Patienten nicht berührt;
- das proximale Einwegventil (220; 920) in einem Fall eines negativen Drucks, definiert als Unterdruck, in Kontakt mit der Trachealwand des Patienten tritt und hiermit einen zusätzlichen Dichtungsabschnitt (272) mit der Trachealwand des Patienten in proximaler Position bereitstellt; und
- das distale Einwegventil (225; 925), in dem Falle eines positiven Drucks, definiert als Überdruck, mit der Trachealwand des Patienten in Kontakt tritt und hiermit einen weiteren zusätzlichen Dichtungsabschnitt (275) mit der Trachealwand des Patienten in distaler Position bereitstellt.

2. Wolkenmanschette (210; 910) nach Anspruch 1, wobei:
das proximale Einwegventil (220; 920) eine konkave Oberfläche an dem proximalen Ende, die auf ein Zentrum der Wolkenmanschette gerichtet ist, umfasst, und
das distale Einwegventil (225; 925) eine andere konkave Oberfläche an dem distalen Ende, die auf das Zentrum der Wolkenmanschette gerichtet ist, umfasst,
derart, dass die konkaven Oberflächen einander zugewandt sind und daher als ihr Spiegelbild erscheinen.

3. Wolkenmanschette (210; 910) nach Anspruch 1 oder 2, wobei das proximale Einwegventil und das distale Einwegventil doppelwandig ist.

4. Wolkenmanschette (210; 910) nach den Ansprüchen 1 bis 3, die eine etwa gleichmäßige Dicke aufweist, wobei insbesondere die Dicke mittels (Blas-)Formen der Wolkenmanschette während deren Fertigung erzielt wird und sie in dem Bereich von 0,05 mm und 0,2 mm liegt, wobei eine Gleichmäßigkeit in dem Bereich von ±5 % liegt.

5. Wolkenmanschette (210; 910) nach den Ansprüchen 1 bis 4, die, durch Auswahl der zu verwendenden Materialien und/oder ihrer Dicke, konfiguriert ist, um auf Manschettendrücke von 5 cm H₂O bis 30 cm H₂O oder Manschettendrücke von bis zu 100 cm H₂O aufgeblasen zu werden.

6. Wolkenmanschette (210; 910) nach den Ansprüchen 1 bis 5, die aus Polyethylenterephthalat (PETP), Polyethylen niedriger Dichte (LDPE), Polyvinylchlorid (PVC), Silikon, Neopren, Polyisopren, Polypropylen oder Polyurethan (PU) hergestellt ist.

7. Wolkenmanschette (210) nach den Ansprüchen 1 bis 6, wobei das proximale Einwegventil (220) zwischen zwei Bereichen (211, 212) bereitgestellt ist.

8. Wolkenmanschette (210) nach den Ansprüchen 1 bis 7, wobei das distale Einwegventil (225) zwischen zwei Bereichen (214, 215) bereitgestellt ist.

9. Wolkenmanschette (210; 910) nach den Ansprüchen 1 bis 8, wobei die Bereiche (211-215; 911-913) in aufgeblasenem Zustand ein(e) insgesamt kugelförmige(s) oder eiförmige(s) Wolkenform oder Volumen ausbilden, oder aber die insgesamte Form oder das insgesamte Volumen in aufgeblasenem Zustand kegelförmig oder kegelstumpfförmig ist.

10. Wolkenmanschette (210; 910) nach den Ansprüchen 1 bis 9, wobei:
der maximale Durchmesser (d3, d5) der Bereiche (211, 212, 214, 215; 911, 913) zuerst räumlich allmählich zunimmt, bis der maximale Durchmesser (d2) des mindestens einen Bereichs (213; 912) erreicht ist, und er dann allmählich wieder abnimmt.

11. Wolkenmanschette (210) nach Anspruch 10, wobei:
das proximale Einwegventil (220) auf den aufblasbaren Manschettenkörper (290) in der Nähe des proximalen Endes (216) zwischen zwei Bereichen (211, 212) bereitgestellt ist; und
das distale Einwegventil (225) auf den aufblasbaren Manschettenkörper (290) in der Nähe des distalen Endes (217) zwischen zwei Bereichen (214, 215) bereitgestellt ist.

12. Endotrachealtubus (ETT)(20, 40)-System zum Beatmen eines Patienten, das ETT-System umfassend:
- eine Wolkenmanschette (210, 410) nach den Ansprüchen 1 bis 11;
- ein oder mehrere Manschettenaufblaslumen; und
- einen Beatmungstubus (200, 400).

13. ETT(20, 40)-System nach Anspruch 12, wobei der Beatmungstubus eine Öffnung (206, 406) zum Aufblasen der Wolkenmanschette über ein Manschettenaufblaslumen (205, 405), das in den Beatmungstubus integriert ist, umfasst.

14. ETT(40)-System nach Anspruch 12 oder 13, wobei die Wolkenmanschette (410) als primäre Wolkenmanschette definiert ist, das ETT-System ferner umfassend:
- eine zusätzliche Wolkenmanschette (450) nach den Ansprüchen 1 bis 11, die als sekundäre Wolkenmanschette in einer distalen Position hinsichtlich der primären Wolkenmanschette definiert ist, wobei die zwei Wolkenmanschetten (410, 450) mit einem oder mehreren Manschettenaufblaslumen versehen sind, um die zwei Manschetten aufzublasen und/oder zu entleeren;
- ein Mittel zum Erfassen und/oder Messen von Luftstromparametern in einer Zwischenmanschettenregion, die die primäre Wolkenmanschette mit der sekundären Wolkenmanschette verbindet; und
- eine Manschettensteuerung, umfassend (i) eine elektronische Vorrichtung zum Eingeben erfasster und/oder gemessener Luftstromparameter und zum Berechnen eines oder mehrerer geregelter Luftströme aus den Luftstromparametern, und (ii) ein oder mehrere erste mechanische Mittel zum Bereitstellen der geregelten Luftströme.

15. ETT(40)-System nach Anspruch 14, ferner umfassend ein nicht flüchtiges maschinenlesbares Speichermedium, das ein Computerprogrammprodukt speichert, das auf einer Verarbeitungsmaschine betriebsfähig ist, zum Ausführen des Berechnens der elektronischen Vorrichtung.

## Revendications

1. Ballonnet en forme de nuage (210 ; 910) pour un système de tube endotrachéal (ETT), le ballonnet en forme de nuage pouvant être attaché à un tube de ventilation du système ETT, et le ballonnet en forme de nuage comprenant : (i) une première partie cylindrique prévue au niveau d'une extrémité proximale (216 ; 916) du ballonnet en forme de nuage et une seconde partie cylindrique prévue au niveau d'une extrémité distale (217 ; 917) du ballonnet en forme de nuage, dans lequel les première et seconde parties cylindriques ont une surface correspondant à une forme cylindrique du tube de ventilation (200 ; 900) ; (ii) une valve unidirectionnelle proximale (220 ; 920) au niveau de l'extrémité proximale du en forme de nuage, la valve unidirectionnelle proximale étant gonflable ; (iii) une valve unidirectionnelle distale (225 ; 925) au niveau de l'extrémité distale du ballonnet en forme de nuage, la valve unidirectionnelle distale étant gonflable ; et (iv) un corps de ballonnet gonflable (290) entre l'extrémité proximale (216 ; 916) et l'extrémité distale (217 ; 917) ;
dans lequel :
- le corps de ballonnet gonflable (290) ayant une forme de nuage avec au moins deux sections ayant une différence de diamètre maximal, les sections (211 à 215 ; 911 à 913) ayant un diamètre changeant progressivement le long de la longueur du tube de ventilation (200 ; 900), lesdites sections comprenant des formes arrondies ayant des surfaces convexes, dans lequel
∘ au moins une section (213 ; 912) fonctionne en tant que partie d'étanchéité (270) adaptée à former une bande infroissable contre la paroi trachéale d'un patient lorsqu'elle est gonflée, et ayant un diamètre maximal (d2) ; et
∘ les au moins deux sections (212, 213 ; 911, 912), ayant une différence de diamètre maximal (d3, d2), comprennent ladite au moins une section (213 ; 912) et une section plus petite (212 ; 911) ayant un diamètre maximal (d3) plus petit que le diamètre maximal (d2) de l'au moins une section (213 ; 912) et dans lequel ladite plus petite section (212 ; 911) ne touche pas la paroi trachéale du patient ;
- la valve unidirectionnelle proximale (220 ; 920) en cas de pression négative définie en tant que sous-pression entrant en contact avec la paroi trachéale du patient, et fournissant ainsi une partie d'étanchéité supplémentaire (272) avec la paroi trachéale du patient en position proximale ; et
- la valve unidirectionnelle distale (225 ; 925) en cas de pression positive définie en tant que surpression entrant en contact avec la paroi trachéale du patient, et fournissant ainsi une autre partie d'étanchéité supplémentaire (275) avec la paroi trachéale du patient en position distale.

2. Ballonnet en forme de nuage (210, 910) selon la revendication 1,
dans lequel :
la valve unidirectionnelle proximale (220 ; 920) comprend une surface concave au niveau de l'extrémité proximale dirigée vers un centre du ballonnet en forme de nuage, et
la valve unidirectionnelle distale (225 ; 925) comprend une autre surface concave au niveau de l'extrémité distale dirigée vers le centre du ballonnet en forme de nuage,
de telle sorte que les surfaces concaves se rapprochent l'une de l'autre et apparaissent donc en tant que leur image miroir.

3. Ballonnet en forme de nuage (210 ; 910) selon la revendication 1 ou 2, dans lequel la valve unidirectionnelle proximale et la valve unidirectionnelle distale sont à double paroi.

4. Ballonnet en forme de nuage (210 ; 910) selon les revendications 1 à 3, ayant une épaisseur approximativement uniforme, en particulier l'épaisseur est obtenue au moyen du moulage (par soufflage) du ballonnet en forme de nuage pendant sa fabrication, et se situe dans la plage comprise entre 0,05 mm et 0,2 mm, avec une uniformité dans la plage de ±5 %.

5. Ballonnet en forme de nuage (210 ; 910) selon les revendications 1 à 4, étant conçu, par sélection des matériaux à utiliser et/ou de son épaisseur, pour être gonflé à des pressions de ballonnet de 5 cm H₂O à 30 cm H₂O ou à des pressions de ballonnets allant jusqu'à 100 cm H₂O.

6. Ballonnet en forme de nuage (210 ; 910) selon les revendications 1 à 5, étant fabriqué à partir de téréphtalate de polyéthylène (PETP), de polyéthylène basse densité (LDPE), de polychlorure de vinyle (PVC), de silicone, de néoprène, de polyisoprène, de polypropylène ou de polyuréthane (PU).

7. Ballonnet en forme de nuage (210) selon les revendications 1 à 6, dans lequel la valve unidirectionnelle proximale (220) est prévue entre deux sections (211, 212).

8. Ballonnet en forme de nuage (210) selon les revendications 1 à 7, dans lequel la valve unidirectionnelle distale (225) est prévue entre deux sections (214, 215).

9. Ballonnet en forme de nuage (210 ; 910) selon les revendications 1 à 8, dans lequel les sections (211 à 215 ; 911 à 913) forment une forme ou un volume global de nuage sphérique ou ovoïde à l'état gonflé, ou bien la forme ou le volume global est conique ou tronconique à l'état gonflé.

10. Ballonnet en forme de nuage (210 ; 910) selon les revendications 1 à 9, dans lequel :
le diamètre maximal (d3, d5) des sections (211, 212, 214, 215 ; 911, 913) augmente d'abord, progressivement dans l'espace, jusqu'à ce que le diamètre maximal (d2) de l'au moins une section (213 ; 912) soit atteint, puis diminue à nouveau progressivement.

11. Ballonnet en forme de nuage (210) selon la revendication 10, dans lequel :
la valve unidirectionnelle proximale (220) est prévue sur le corps de ballonnet gonflable (290) à proximité de l'extrémité proximale (216), entre deux sections (211, 212) ; et
la valve unidirectionnelle distale (225) est prévue sur le corps de ballonnet gonflable (290) à proximité de l'extrémité distale (217), entre deux sections (214, 215).

12. Système de tube endotrachéal (ETT) (20, 40) permettant de ventiler un patient, le système ETT comprenant :
- un ballonnet en forme de nuage (210, 410) selon les revendications 1 à 11 ;
- une ou plusieurs lumières de gonflage de ballonnet ; et
- un tube de ventilation (200, 400).

13. Système ETT (20, 40) selon la revendication 12, dans lequel le tube de ventilation comprend une ouverture (206, 406) permettant de gonfler le ballonnet en forme de nuage par l'intermédiaire d'une lumière de gonflage de ballonnet (205, 405) intégrée dans le tube de ventilation.

14. Système ETT (40) selon la revendication 12 ou 13, dans lequel le ballonnet en forme de nuage (410) est défini en tant que ballonnet en forme de nuage primaire, le système ETT comprenant en outre :
- un ballonnet en forme de nuage supplémentaire (450) selon les revendications 1 à 11, défini en tant que ballonnet en forme de nuage secondaire dans une position distale par rapport à un ballonnet en forme de nuage primaire, dans lequel les deux ballonnets en forme de nuage (410, 450) sont pourvus d'une ou plusieurs lumières de gonflage de ballonnet permettant de gonfler et/ou dégonfler les deux ballonnets ;
- un moyen permettant de détecter et/ou mesurer des paramètres de flux d'air dans une région entre les ballonnets reliant un ballonnet en forme de nuage primaire à un ballonnet en forme de nuage secondaire ; et
- un dispositif de commande de ballonnet comprenant (i) un dispositif électronique permettant d'entrer des paramètres de flux d'air détectés et/ou mesurés, et de calculer un ou plusieurs flux d'air régulés à partir des paramètres de flux d'air, et (ii) un ou plusieurs premiers moyens mécaniques permettant de fournir les flux d'air régulés.

15. Système ETT (40) selon la revendication 14, comprenant en outre un support de stockage non transitoire lisible par machine stockant un produit-programme d'ordinateur, utilisable sur un moteur de traitement, pour exécuter ledit calcul du dispositif électronique.
